# EUROPEAN PATENT APPLICATION

(11) **EP 4 056 270 A1**
(43) Date of publication of application: **14.09.2022**
(21) Application number: 20883990.2
(22) Date of filing: 04.11.2020
(51) Int. Cl.: B01L 3/00

(54) **DROPLET DEFORMATION-BASED METHOD OF TRANSFERRING MATERIAL INTO CELLS AND CHIP FOR SAME**

(30) Priority: 06.11.2019 KR 20190140657; 07.10.2020 KR 20200129659
(71) Applicant: Korea University Research and Business Foundation, Seoul 02841 (KR)
(72) Inventor: CHUNG, Aram, Seoul 04425 (KR); JOO, Byeong Ju, Seoul 02853 (KR); LEE, Ha Sung, Seoul 05224 (KR)
(74) Representative: Zech, Stefan Markus
(86) International application number: PCT/KR2020/015261
(87) International publication number: WO 2021/091205

(57) **Abstract**

Provided is a method of transferring a material into cells, comprising the steps of: forming droplets consisting of a material to be transferred and cells; and a step of subjecting the formed droplets to deformation, thereby transferring the material to be transferred, into the cells.

## Description

### Field

The present disclosure relates to a droplet deformation-based method for delivering a material into cells and a chip for the same, and more specifically, to a droplet deformation-based method for delivering a material into cells and a chip for the same that improve efficiency, economic feasibility, and the like by implementing the delivery of the material into the cells using microdroplets containing a hydrophilic solution containing the cells.

### Description of Related Art

Delivery of a material into cells is one of the most basic experiments in cell engineering. The material is usually delivered using a carrier or by defining nanopores in a cell membrane/nuclear membrane. In viral or Lipofectamine-centered carrier techniques, high-efficiency material delivery is possible when the technique is optimized, but there are problems such as safety, slow delivery velocity, labor/cost-intensive carrier preparation process, low reproducibility, and the like.

On the other hand, methods (e.g., an electroporation or a microneedle) for defining the nanopores by applying energy to the cell membrane are relatively advantageous as various materials are able to be delivered to various cell lines. However, low cell viability, denaturation of the delivered material, and low throughput resulted from invasiveness of the method are pointed out as major limitations. To solve such problems, use of microfluidic devices capable of treating a large amount of cells is remarkable. Typically, there is a platform that creates a narrow constriction or a bottleneck in a microtubule and defines the nanopores in the cell membrane through physical deformation of the cells that occurs when the cells pass through the bottleneck. However, such approach has major drawbacks such as clogging of the bottleneck itself, inconsistent material delivery efficiency, and the like during the experiment.

For example, US Patent No. 2014-0287509 (hereinafter, referred to as prior art) discloses a technique for inducing the cell deformation by applying a pressure to the cells by allowing the cells to directly flow through a channel having a bottleneck structure. However, in this case, there is a problem in that the cells clog the bottleneck structure. In addition, the cells are directly in contact with a channel wall to be subjected to stress resulted from friction or the like, so that the cell viability is lowered and the channel clogging occurs. In addition, there are problems that the material delivery is non-uniform because of the non-uniform cell deformation resulted from non-uniform velocity, and the material delivery efficiency is fundamentally low because the delivery occurs only through a diffusion phenomenon. Therefore, it is urgent to develop an innovative next-generation platform for the delivery of the material into the cells that may deliver the various materials uniformly and with high efficiency into the cells while maintaining the great treatment function of the microfluidic device.

### DISCLOSURE

### TECHNICAL PURPOSES

Therefore, a purpose of the present disclosure is to provide a method and a system that greatly improve a delivery efficiency and a system stability with a material delivery scheme using microdroplets.

### TECHNICAL SOLUTIONS

One aspect of the present disclosure provides a method for delivering a material into cells including generating droplets composed of the material to be delivered and the cells, and deforming the generated droplets to deliver the material to be delivered into the cells.

In one implementation, the droplets are generated by allowing a fluid of a first phase containing the cells and the material to flow to a fluid of a second phase immiscible with the fluid of the first phase.

In one implementation, the deforming of the droplets includes reducing a diameter of a channel.

In one implementation, the deforming of the droplets includes generating vortices in a fluid where the droplets flow.

Another aspect of the present disclosure provides a platform for delivering a material into cells including a channel for allowing droplets containing the material to be delivered and the cells to flow therethrough, and deformation means for physically deforming the droplets flowing through the channel to deliver the material to be delivered into the cells.

In one implementation, the deformation means is a microchannel having a reduced diameter or a junction where vortices are able to occur due to a collision of fluids.

Still another aspect of the present disclosure provides a method for delivering a material into cells including generating droplets of a hydrophilic solution containing the cells and the material to be delivered in a hydrophobic solution, allowing the hydrophobic solution containing the generated droplets to flow through a channel, primarily deforming the droplets by allowing the hydrophobic solution flowing through the channel to pass through another channel having a smaller diameter than the channel, and secondarily deforming the droplets in the channel after the droplets pass through said another channel, wherein, in the primary deforming and the secondary deforming of the droplets, the cells are not in direct contact with a wall of the channel.

In one implementation, in the primary deforming of the droplets, the droplets are squeezed, and at this time, nanopores are defined in a cell membrane of the cells in the droplets. In one implementation, the secondary deforming of the droplets, the droplets are restored to a shape before the primary deforming of the droplets, and at this time, the material to be delivered is delivered into the cells through pores of a cell membrane defined in the primary deforming of the droplets.

In one implementation, in the secondary deforming of the droplets, diffusion and convection occur in the droplets, so that a delivery efficiency of the material to be delivered in the droplets into the cells increases. In one implementation, the droplets pass through said another channel at least once.

In one implementation, the generating of the droplets of the hydrophilic solution containing the cells and the material to be delivered in the hydrophobic solution includes generating the droplets by allowing the hydrophobic solution in a second direction to flow to the hydrophilic solution flowing in a first direction such that the hydrophilic solution and the hydrophobic solution come into contact with each other. In one implementation, the method further includes accelerating the hydrophobic solution having the droplets generated therein by allowing another hydrophobic solution to flow to the hydrophobic solution having the droplets generated therein.

In one implementation, the material to be delivered is at least one selected from a group consisting of a nucleic acid, a protein, a transcription factor, a vector, plasmid, a genetic scissor material, and a nanoparticle.

Still another aspect of the present disclosure provides a microfluidic chip for implementing the method for delivering the material into the cells described above.

In one implementation, the microfluidic chip includes a plurality of channels for implementing the method for delivering the material into the cells described above. In one implementation, the channels are defined in parallel with each other.

### TECHNICAL EFFECTS

The present disclosure implements the delivery of the material into the cells using the microdroplets containing the hydrophilic solution containing the cells. In this case, the uniform and high-efficiency material delivery into the cells is possible by deforming the cells in the droplets by passing the cells in the microdroplets through the bottleneck or the vortex occurrence point with the reduced diameter at the constant velocity. In addition, it is possible to fundamentally prevent the cell surface from coming into direct contact with the microtubule wall face, thereby fundamentally avoiding the channel clogging phenomenon of the prior art and reducing the stress caused by the friction between the cells and the channel wall. Furthermore, compared to the conventional scheme in which the material is delivered only by the diffusion, in the present disclosure, the high delivery efficiency may be secured as the external material may be delivered through the secondary flow in the microdroplets, which occurs when the cell is restored to the original shape thereof, and the exchange, through the diffusion and the convection, of the fluids with the cell membrane interposed therebetween. In addition, economic feasibility may be secured by making the material delivered into the cells into the microdroplets, and the amount of delivered material may also be controlled through the droplet volume.

Therefore, the method and the system for delivering the material into the cells according to the present disclosure may be applied to all research fields and industries related to cell engineering to implement the material delivery into the cells with an inexpensive and simple scheme with high efficiency and high throughput.

### BRIEF DESCRIPTION OF THE DRAWINGS

FIG. 1 is a diagram illustrating a droplet change process using a cell delivery platform having a junction according to an embodiment of the present disclosure.
FIG. 2 is a diagram illustrating a droplet change process using a cell delivery platform having a '+'-shaped junction according to an embodiment of the present disclosure.
FIG. 3 is a flowchart of a method for delivering a material into cells according to an embodiment of the present disclosure.
FIGS. 4 to 7 are schematic diagrams of a method and a system for delivering a material into cells according to an embodiment of the present disclosure.
FIG. 8 is a schematic diagram of a method for delivering a material into a cell according to FIGS. 4 to 7 and a channel photograph of each step.
FIG. 9 shows photographs taken after the lapse of the same time for experimental cases of the prior art and the present disclosure.
FIG. 10 shows results of a microtubule clogging experiment of the prior art and the present disclosure.
Left and right sides of FIG. 11 are results of comparing efficiencies of material delivery into the cells of the present disclosure and the prior art with each other.
FIG. 12 is a photograph of a channel in which the primary deformation and the secondary deformation are continuously repeated according to an embodiment of the present disclosure.
FIG. 13 shows results of the cell encapsulation in the microdroplets, the deformation in the bottleneck section, and FITC-Dextran delivery.
FIG. 14 is a FITC fluorescence signal measurement result for each size of Dextran, and FIG. 15 is a result of comparing viabilities and delivery efficiencies based on cell line types.
FIG. 16 is an experimental result of comparing delivery efficiencies based on a structure and the number of bottleneck channels, and a flow velocity.
FIG. 17 is a result of an experiment on mRNA delivery and GFP expression resulted therefrom for a K562 cell.
FIG. 18 is a result of an mRNA delivery efficiency comparison experiment of the present disclosure (DS), the electroporation (a neon transfection system, EP), which is the most commonly used in the field, and lipofectamine (LP).

### DETAILED DESCRIPTIONS

Hereinafter, a method for delivering a material into cells and a chip therefor according to the present disclosure will be described with reference to the accompanying drawings and embodiments. Unless otherwise defined, all terms in this specification have the same general meaning as understood by those of ordinary skill in the art to which the present disclosure belongs. In case the general meaning of the term conflicts with the meaning of the term used herein, the meaning of the term is based on the definition used herein. In addition, detailed description of the known function and component that may unnecessarily obscure the gist of the present disclosure will be omitted. Throughout the specification, when a component "includes" a certain component, it means that other elements may be further included, rather than excluding other elements, unless otherwise stated.

The present disclosure encapsulates cells and a material to be delivered using droplets and induces physical deformation of the generated droplets using vortices or a channel structure with a reduced diameter to generate micropores in the cells in the droplets, thereby delivering the material.

In one embodiment of the present disclosure, the droplets are generated by mixing, in the channel, a fluid (a first phase) containing the cells and the material to be delivered and an immiscible delivery fluid (a second phase) with each other. In this case, the material to be delivered and the cells are encapsulated in microdroplets, thereby maximizing a delivery efficiency while minimizing an amount of use of the material to be delivered.

A first reason for the use of the droplets in the present disclosure is that a very small volume (~100 pL/cell) of liquid droplets may be used. In one embodiment of the present disclosure, the amount of material to be delivered is determined depending on a volume of hydrophilic droplets excluding a hydrophobic oil phase volume, so that there is an advantage that only a very small amount of material to be delivered may be used.

In addition, because of the very small droplet volume, the encapsulated cells are placed in a high-concentration material to be delivered environment. When the droplets pass through a section that deforms the droplets, such as a bottleneck or a vortex, the material to be delivered of the high concentration is effectively delivered into the cells because of a secondary flow occurred in the droplets, thereby achieving high delivery efficiency. This enables delivery of a macromolecule (e.g., a larger nanoparticle and a plasmid DNA) into the cells, which was not possible in the prior art.

Furthermore, cell-squeezing, which is the prior art, in which the cells themselves flow into the bottleneck section, has a problem in that it is difficult to achieve a uniform delivery because of a large tube clogging phenomenon in the bottleneck and different velocities of the cells of low weight in the channel. However, the present disclosure may minimize the channel clogging phenomenon using the droplets to encapsulate various debris that clogs the microtubule in the droplets. In addition, the clogging phenomenon may also be minimized because of a difference in hydrophilicity/hydrophobicity between the droplets and the microtubule wall, and the uniform delivery may be achieved because of a constant velocity resulted from a weight/volume of the droplets.

Hereinafter, the present disclosure will be described in more detail through one embodiment of the present disclosure. However, the scope of the present disclosure is not limited thereto.

In one embodiment of the present disclosure, an experiment was conducted on a K562 cell line for a possible use in cancer immunotherapy in the future. 2,000 kDa FITC-dextran simulating a large protein was used as the material to be delivered, and a microfluidic chip was made using general photolithography and soft lithography methods.

A droplet-based platform for delivering a material into cells according to one embodiment of the present disclosure delivers the material into the cells through three steps: 1) droplet generation (cell encapsulation), 2) droplet deformation (delivery of the material into the cells), and 3) cell recovery.

In the present disclosure, the deformation of the droplet is a physical scheme, which may be a scheme using the vortices or the channel diameter.

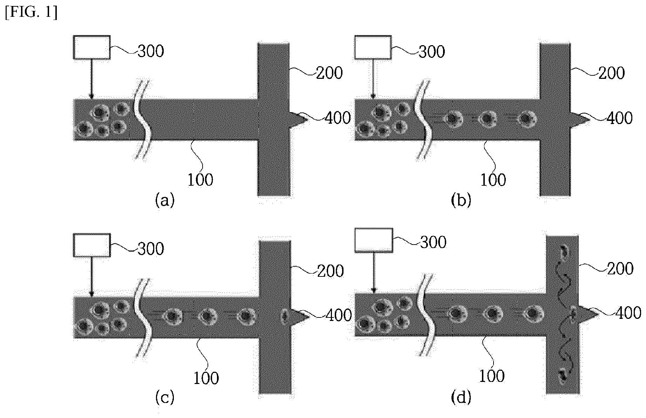

FIG. 1 is a diagram illustrating a droplet change process using a cell delivery platform having a junction according to an embodiment of the present disclosure. In the present disclosure, the 'junction' means a merge point of fluid channels where the vortices may be generated as fluids in different directions collide with each other. A shape of the junction may be a T-shape, a +-shape, a Y-shape, or a combination thereof, and the scope of the present disclosure is not limited to a specific shape.

Referring to FIG. 1, the cell delivery platform according to one embodiment of the present disclosure includes the junction where the vortices are generated by the collision between the fluids or a collision between the fluid and the channel wall.

In FIG. 1, a first channel 100 for defining a path through which the fluid containing the droplets flows; a second channel 200 located at an end of the first channel 100 and extending vertically from both side faces of the first channel 100; and cell acceleration means 300 disposed in the first channel 100 to control a fluid velocity in the first channel 100 are included. That is, in FIG. 1, an intersection in the 'T' shape becomes the junction and the vortices are generated as the fluid and the channel wall collide with each other at the junction.

In one embodiment of the present disclosure, a flow velocity and inertia of the fluid passing through the first channel 100 are controlled via the cell acceleration means 300 to induce vortex generation and vortex breakdown at and near the junction, thereby inducing the cell delivery with high efficiency.

More specifically, the present disclosure may induce droplet injection-droplet alignment-collision-cell deformation-material delivery using only the inertia and inertial flow, thereby, without using separate active cell delivery means and a vector such as a virus, delivering various materials (e.g., a genetic scissor material, a plasmid, and the like) into the cells in large quantities (equal to or more than millions per minute) through nanopores defined in the cell membrane.

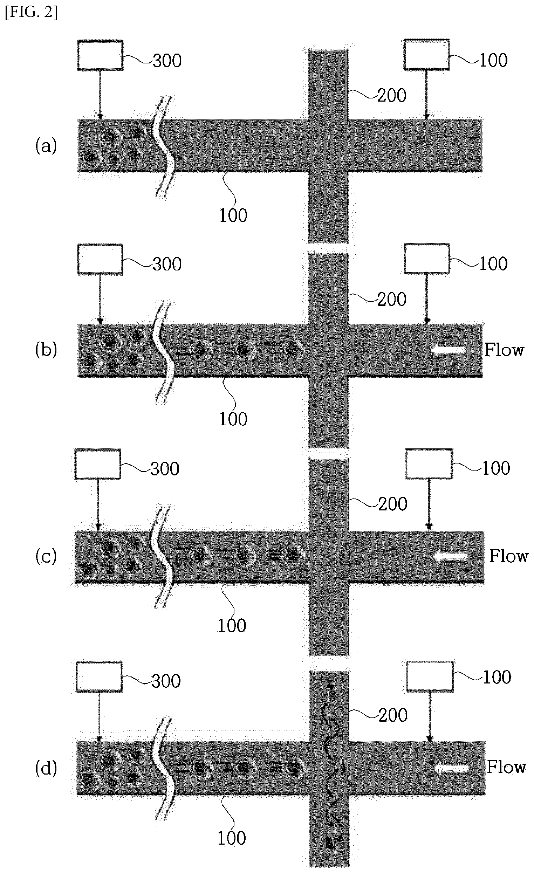

FIG. 2 is a diagram illustrating a droplet change process using a cell delivery platform having a '+'-shaped junction according to an embodiment of the present disclosure.

Referring to FIG. 2, a cell delivery platform having a '+'-shaped junction includes a first channel 100 through which a fluid containing droplets flows; a second channel 200 vertically intersecting the first channel 100; and first cell acceleration means 300 disposed on one side of the first channel 100 to control a fluid velocity in the first channel 100 in a first direction.

According to one embodiment of the present disclosure, the fluid in the first channel 100 flows towards a point where the first channel 100 vertically intersects with the second channel 200, and the first cell acceleration means 300 applies, to cells on the first channel 100, a kinetic energy capable of deforming the cell membrane at a level at which the nanopores are defined in the cell membrane of the cell by the vortices generated at the junction where the first channel 100 and the second channel 200 vertically intersect each other.

In addition, second cell acceleration means 300' for controlling the fluid velocity in the first channel 100 in a second direction may be further included on the other side of the first channel 100.

In particular, the vortices are generated at the point where the first channel 100 and the second channel 200 vertically intersect each other by the first and second cell acceleration means 300 and 300' controlling the fluid velocity in opposite directions in the first channel 100. Because of an inertial force and the inertial flow resulted therefrom, the cell may be physically deformed to deform the cell membrane.

Similar to the above-mentioned 'T'-shaped cell delivery platform, the '+'-shaped cell delivery platform may induce droplet injection-droplet alignment-collision-cell deformation-material delivery using only the inertia and the inertial flow, thereby, without using the separate active cell delivery means, delivering the various materials (e.g., the genetic scissor material, the plasmid, and the like) into the cells in large quantities (equal to or more than millions per minute) through the nanopores. In addition, due to the vortex breakdown that occurs after the vortex generation, the cells in the droplets are deformed secondarily, so that the material to be delivered in the droplets is delivered into the cells.

In another embodiment of the present disclosure, unlike the conventional squeezing scheme, droplets of a hydrophilic solution containing the cells and the material to be delivered are first generated in a hydrophobic solution such as oil, and then, the droplets pass through a channel section (a compression hole or the bottleneck section) where the diameter decreases to deliver the material into the cells. As a result, the external material may be delivered by diffusion and convection through a secondary flow in the microdroplets and an exchange between fluids with the cell membrane interposed therebetween during recovery to an original shape of the cell.

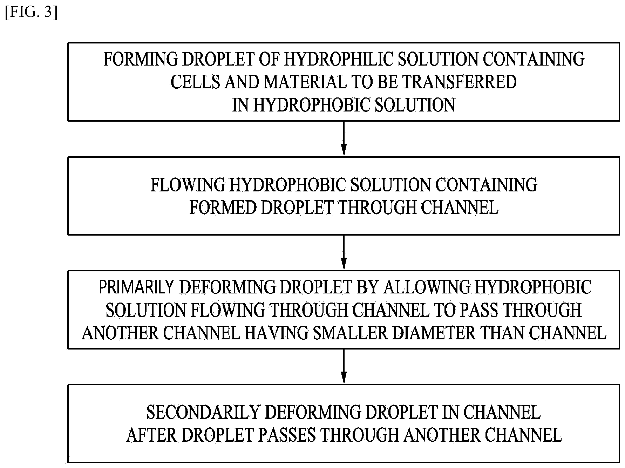

FIG. 3 is a flowchart of a method for delivering a material into cells according to an embodiment of the present disclosure.

Referring to FIG. 3, the method for delivering the material into the cells according to an embodiment of the present disclosure includes: generating the droplets of the hydrophilic solution containing the cells and the material to be delivered in the hydrophobic solution; allowing the hydrophobic solution containing the generated droplets to flow through the channel; primarily deforming the droplets by allowing the hydrophobic solution flowing through the channel to pass through another channel having a smaller diameter than the channel; and secondarily deforming the droplets in the channel after the droplets pass through said another channel. In the primary deforming and the secondary deforming of the droplets, the cells are not in direct contact with the channel wall.

Therefore, the present disclosure may fundamentally avoid the channel clogging phenomenon of the prior art by fundamentally preventing a cell surface from being in direct contact with a channel (microtubule) wall face, and may reduce stress resulted from the cell-wall friction or the like.

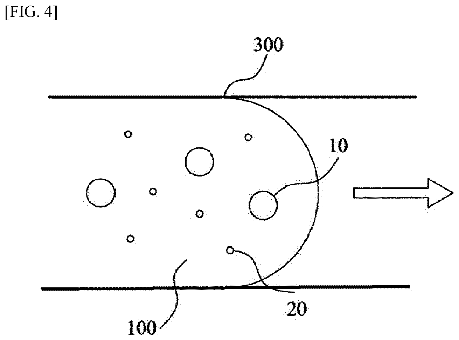

FIGS. 4 to 7 are schematic diagrams of a method and a system for delivering a material into cells according to an embodiment of the present disclosure.

Referring to FIG. 4, in the method for delivering the material into the cells according to the present disclosure, a hydrophilic solution (e.g., aqueous solution) 100 containing a material to be delivered 20, which is a material to be delivered into a cell, and a cell 10 flows in a channel 300 in the first direction.

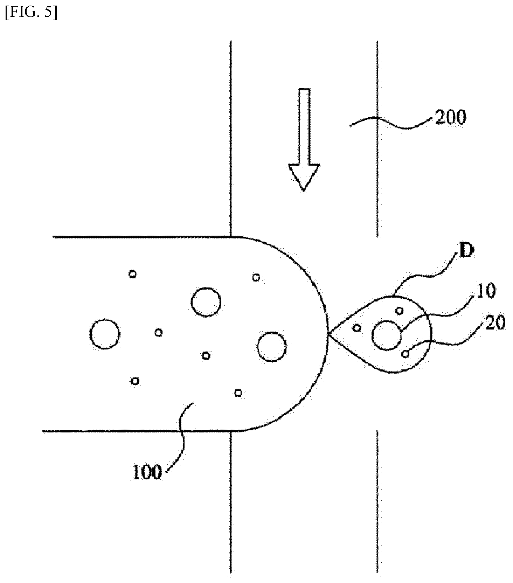

Referring to FIG. 5, thereafter, a hydrophobic solution 200 such as oil flows to the hydrophilic solution 100 in the second direction at a high velocity. Accordingly, as the hydrophilic solution 100 collides and is mixed with the hydrophobic solution 200, a droplet D of the hydrophilic solution (containing the cell and the material to be delivered) is generated in the hydrophobic solution 200 and flows in the channel 100 (a flow-focusing scheme).

In this case, unlike the prior art of allowing the material to be delivered to flow to the entire fluid, the present disclosure does not need to use an additional material to be delivered in response to an increase in flow velocity, thereby remarkably reducing an amount of use of the external material to be delivered.

When necessary, the droplet D may be accelerated to have an enough flow velocity by injecting a hydrophobic solution into and allowing the hydrophobic solution to flow to the hydrophobic solution containing the hydrophilic droplet again at a velocity v2 higher than an initial velocity v1.

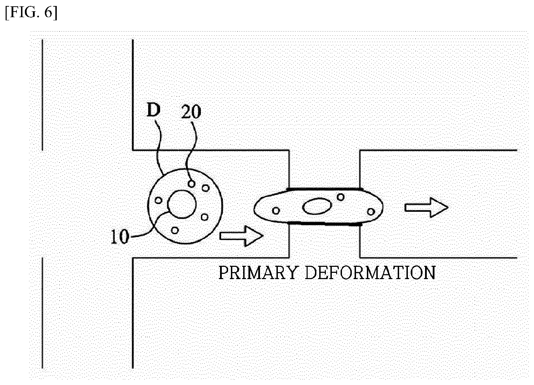

Referring to FIG. 6, the droplet D passes through another channel section (a bottleneck section) 110 having a smaller diameter than the channel 100 at a constant velocity from the channel 100, so that the droplet D is primarily deformed.

In the primary deformation step, the droplets are squeezed. At this time, nanopores are defined in the cell membrane of the cell in the droplets, so that the material to be delivered is delivered into the cell.

In contrast to the prior art, which puts the cells in direct contact with the narrowing bottleneck channel wall, the present disclosure allows the droplets that encapsulate the cells and the material to be delivered to flow in the hydrophobic solution to prevent the cells from being in direct contact with the channel wall, thereby preventing the clogging in the narrowing bottleneck channel and reducing the stress caused by the contact between the cell and channel wall.

In addition, in the prior art, the cells approach at different velocities within the microchannel and pass through the bottleneck section, causing the cell membrane to open, so that the external material delivery is non-uniform. However, in the present disclosure, the cells are encapsulated in the droplets and flow at a center of the microtubule, so that the cells flow and deform at a constant velocity, which is equal to a droplet velocity. Thus, the present disclosure has the uniform delivery efficiency.

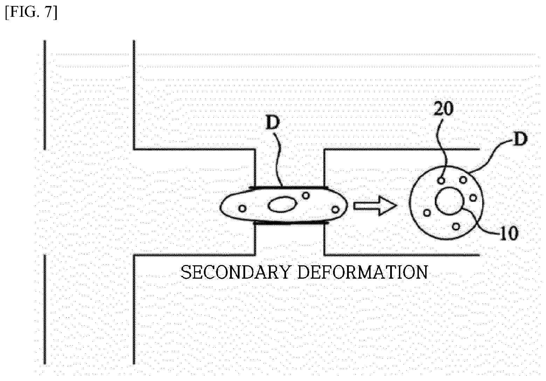

Referring to FIG. 7, the droplet that has passed through said another channel 110 with the reduced channel diameter flows into the channel 100 again. At this time, the secondary deformation occurs in the droplet D and the cell 10, so that the cell is restored to an original size thereof. Herein, the 'original size' refers to a size of the cell in a state of not being physically deformed by at least the bottleneck section.

Based on the secondary deformation, the external material is delivered by the diffusion and the convection through the secondary flow in the microdroplets and the exchange between the fluids with the cell membrane interposed therebetween. As a result, the delivery efficiency is greatly improved by a space between the material to be delivered and the cells concentrated in the form of droplet, which will be described in more detail below.

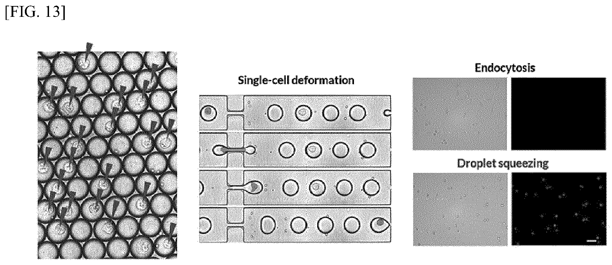

In another embodiment of the present disclosure, each of the primary deformation and the secondary deformation may be performed at least twice. For this purpose, a plurality of other channels may be disposed in one channel (see FIG. 13).

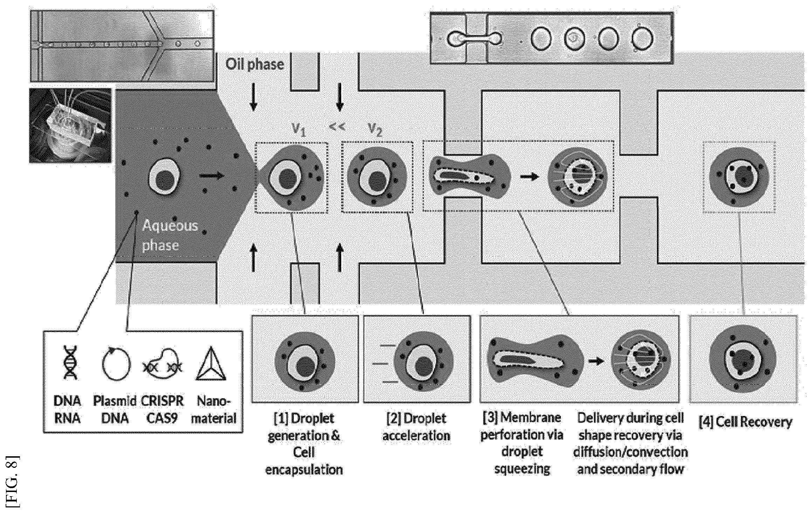

FIG. 8 is a schematic diagram of a method for delivering a material into a cell according to FIGS. 4 to 7 and a channel photograph of each step.

Referring to FIG. 8, by allowing the cells and the material to be delivered to flow through the microfluidic chip, excellent delivery of the material into the cells may be implemented without using artificial cell invasive means such as separate electroporation. In addition, when the hydrophobic solution such as the oil is injected with the velocity v2 higher than the initial injection velocity v1 after the droplets are generated, the droplets may be accelerated to have a desired velocity. The method for delivering the material into the cells according to the present disclosure may be implemented in the chip in which the microchannel is defined. In this case, there is an advantage that a separate micro-element (e.g., a microneedle for a purpose of perforation or the like) is not required.

### Experimental Example

### Flow velocity comparison

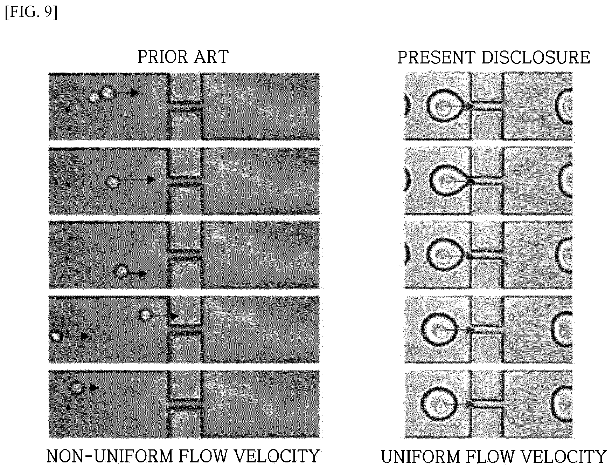

FIG. 9 shows photographs taken after the lapse of the same time for experimental cases of the prior art and the present disclosure.

Referring to FIG. 9, it may be seen that cell positions are different because of different flow velocities in the prior art. However, it may be seen that the present disclosure shows a constant and uniform flow velocity.

In other words, in the prior art, the flow velocity is non-uniform because the cells flow in the channel by being spread widely. However, the droplets according to the present disclosure are generated at the center of the channel, which means that the droplets flow with very uniform shape and velocity. This means that, in the case of the present disclosure, which encapsulates the cells in the form of the droplet, there is virtually no change in the flow velocity despite a microfluid behavior. This in turn results in the uniform material delivery into the cells.

### Microtubule clogging comparison

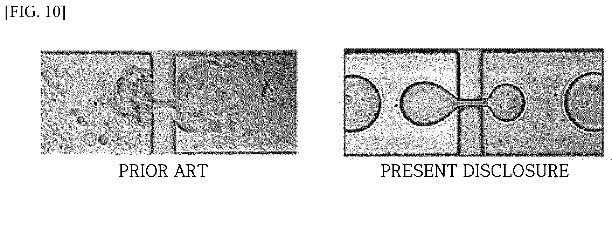

FIG. 10 shows results of a microtubule clogging experiment of the prior art and the present disclosure.

Referring to FIG. 10, in the prior art in which the cell passes through the bottleneck channel by being in direct contact with the channel wall, the cell membrane comes into direct contact with the microtubule wall, resulting in the channel clogging. However, in the present disclosure, the cell does not directly contact the wall face through the use of the microdroplets, so that the channel clogging is minimized, which may be seen from the results of FIG. 8.

### Delivery efficiency and viability comparison

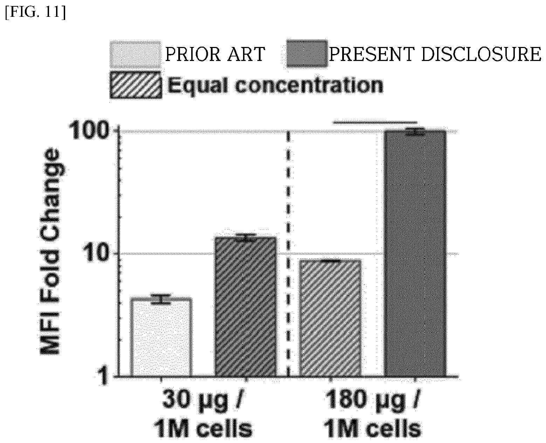

Left and right sides of FIG. 11 are results of comparing efficiencies of material delivery into the cells of the present disclosure and the prior art with each other.

Referring to FIG. 11, compared to the prior art in which the material delivery into the cells occurs only by the diffusion, the present disclosure shows the efficiency higher than that of the prior art because of the delivery of the external material by the diffusion and the convection, and the additional secondary flow that occurs after the microdroplets pass through the bottleneck.

In particular, the method for delivering the material into the cell according to the present disclosure has good efficiency for the same amount, and the efficiency is much better at the same concentration. This is because a possibility of exchange between the cell and the material to be delivered by the diffusion and the convection increases as the two-step deformation occurs in the narrow droplets in the state in which the material to be delivered and the cells are confined in the narrow droplets.

### Delivery efficiency experiment for various immune cell lines

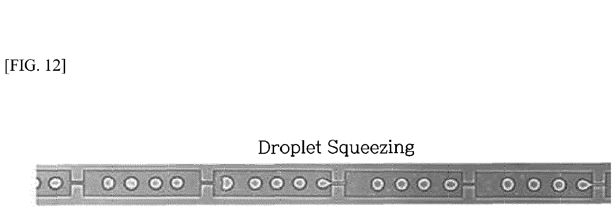

FIG. 12 is a photograph of a channel in which the primary deformation and the secondary deformation are continuously repeated according to an embodiment of the present disclosure.

Referring to FIG. 12, it is possible to increase the efficiency of the material delivery into the cells by continuously constituting the bottleneck sections with the reduced diameter in one channel.

FIG. 13 shows results of the cell encapsulation in the microdroplets, the deformation in the bottleneck section, and FITC-Dextran delivery.

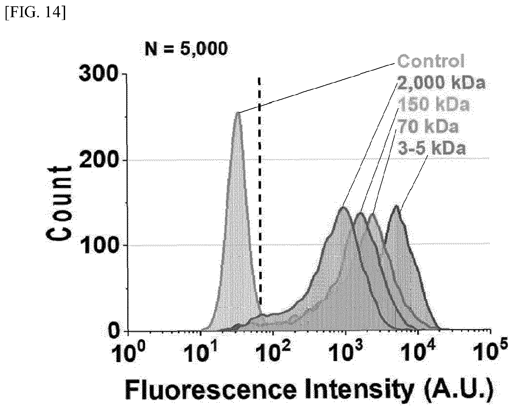

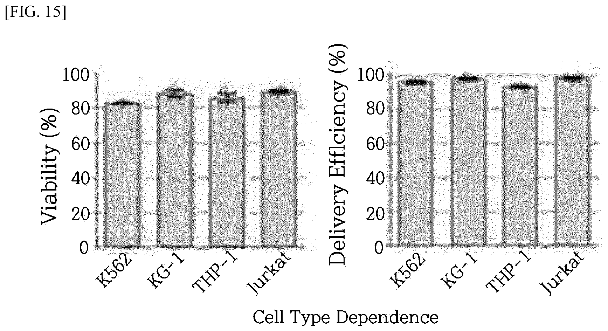

FIG. 14 is a FITC fluorescence signal measurement result for each size of Dextran, and FIG. 15 is a result of comparing viabilities and delivery efficiencies based on cell line types.

Referring to FIGS. 14 and 15, it may be seen that the viabilities are equal to or higher than 80 % and the delivery efficiencies reach 99 %.

### Comparison of delivery efficiencies based on channel structure and flow velocity

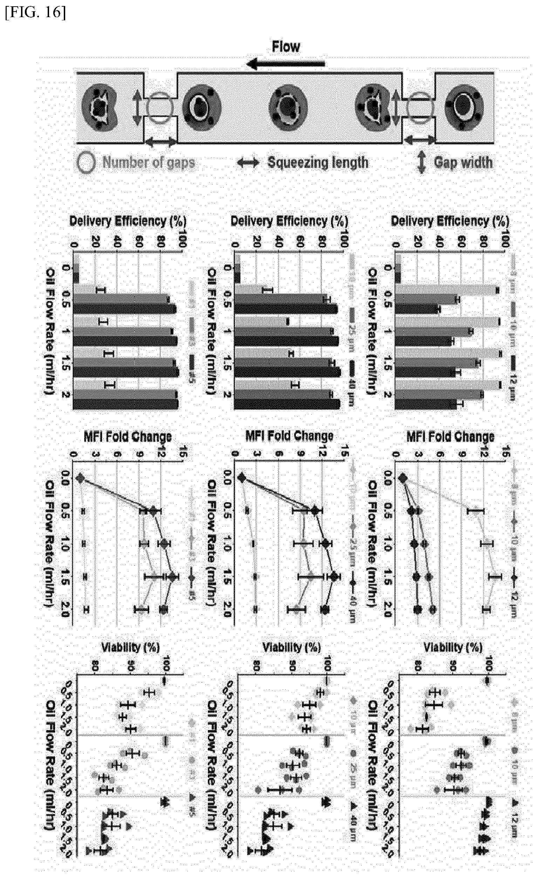

FIG. 16 is an experimental result of comparing delivery efficiencies based on a structure and the number of bottleneck channels, and a flow velocity.

Referring to FIG. 16, it may be seen that, as the flow velocity increases to a level equal to or higher than a certain level, the efficiency of the material delivery into the cells increases, but the efficiency does not increase any more later and the viability is also lowered because the cell does not revert to the original shape thereof. In addition, it may be seen that the smaller the diameter of the bottleneck channel section, the higher the delivery efficiency, but the lower the viability. In addition, it may be seen that as the number of bottleneck sections increases, the delivery efficiency increases, but the viability is also lowered.

### K562 cell-targeted experiment

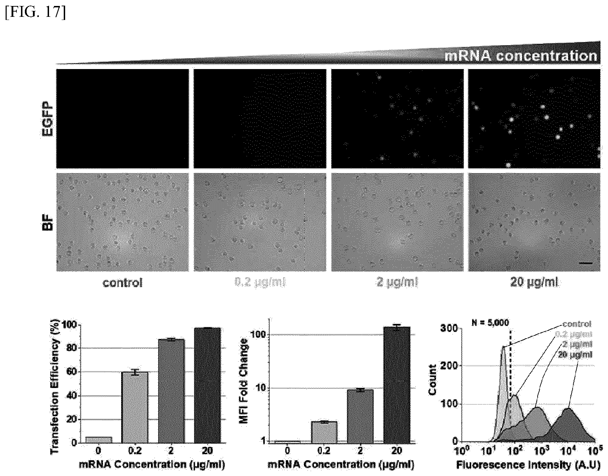

FIG. 17 is a result of an experiment on mRNA delivery and GFP expression resulted therefrom for a K562 cell.

Referring to FIG. 17, the higher the mRNA delivery amount, the higher the efficiency. Thus, when the present disclosure is used, it may be seen that the mRNA delivery and the GFP expression resulted therefrom may be effectively induced for the K562 cell.

### Comparison with conventional delivery method

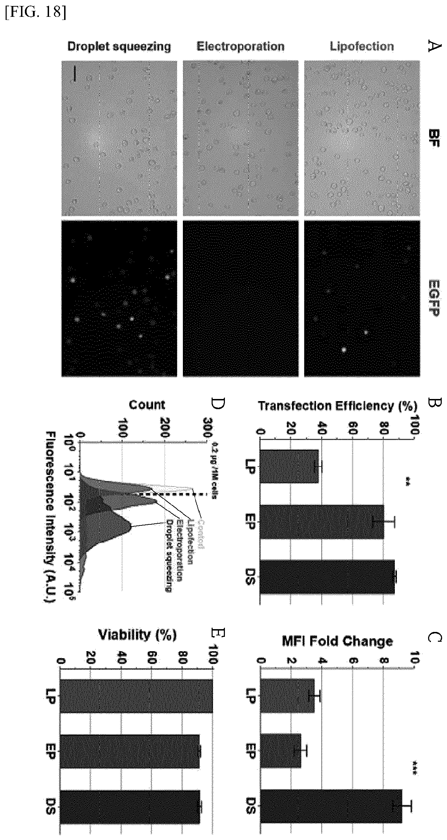

FIG. 18 is a result of an mRNA delivery efficiency comparison experiment of the present disclosure (DS), the electroporation (a neon transfection system, EP), which is the most commonly used in the field, and lipofectamine (LP).

Referring to FIG. 18, it may be seen that the present disclosure exhibits significantly higher expression efficiency compared to the prior art.

The delivery method according to the present disclosure may be implemented through the structure of the microfluid channel. The chip realizing such a microfluid channel also falls within the scope of the present disclosure. In order to obtain a higher production yield, the plurality of above-described channels may be defined in the chip, and the channels may be defined in series or in parallel. All of these are within the scope of the present disclosure.

## Claims

1. A method for delivering a material into cells, the method comprising:
generating droplets composed of the material to be delivered and the cells; and
deforming the generated droplets to deliver the material to be delivered into the cells.

2. The method of claim 1, wherein the droplets are generated by allowing a fluid of a first phase containing the cells and the material to flow to a fluid of a second phase immiscible with the fluid of the first phase.

3. The method of claim 1, wherein the deforming of the droplets includes:
reducing a diameter of a channel.

4. The method of claim 1, wherein the deforming of the droplets includes:
generating vortices in a fluid where the droplets flow.

5. A platform for delivering a material into cells, the platform comprising:
a channel for allowing droplets containing the material to be delivered and the cells to flow therethrough; and
deformation means for physically deforming the droplets flowing through the channel to deliver the material to be delivered into the cells.

6. The platform of claim 5, wherein the deformation means is a microchannel having a reduced diameter.

7. The platform of claim 6, wherein the deformation means is a junction where vortices are able to occur due to a collision of fluids.

8. A method for delivering a material into cells, the method comprising:
generating droplets of a hydrophilic solution containing the cells and the material to be delivered in a hydrophobic solution;
allowing the hydrophobic solution containing the generated droplets to flow through a channel;
primarily deforming the droplets by allowing the hydrophobic solution flowing through the channel to pass through another channel having a smaller diameter than the channel; and
secondarily deforming the droplets in the channel after the droplets pass through said another channel,
wherein, in the primary deforming and the secondary deforming of the droplets, the cells are not in direct contact with a wall of the channel.

9. The method of claim 8, wherein, in the primary deforming of the droplets, the droplets are squeezed, and at this time, nanopores are defined in a cell membrane of the cells in the droplets.

10. The method of claim 8, wherein, in the secondary deforming of the droplets, the droplets are restored to a shape before the primary deforming of the droplets, and at this time, the material to be delivered is delivered into the cells through pores of a cell membrane defined in the primary deforming of the droplets.

11. The method of claim 10, wherein, in the secondary deforming of the droplets, diffusion and convection occur in the droplets, so that a delivery efficiency of the material to be delivered in the droplets into the cells increases.

12. The method of any one of claims 8 to 11, wherein the droplets pass through said another channel at least once.

13. The method of claim 8, wherein the generating of the droplets of the hydrophilic solution containing the cells and the material to be delivered in the hydrophobic solution includes:
generating the droplets by allowing the hydrophobic solution in a second direction to flow to the hydrophilic solution flowing in a first direction such that the hydrophilic solution and the hydrophobic solution come into contact with each other.

14. The method of claim 8, further comprising:
accelerating the hydrophobic solution having the droplets generated therein by allowing another hydrophobic solution to flow to the hydrophobic solution having the droplets generated therein.

15. The method of claim 8, wherein the material to be delivered is at least one selected from a group consisting of a nucleic acid, a protein, a transcription factor, a vector, plasmid, a genetic scissor material, and a nanoparticle.

16. A microfluidic chip for implementing the method for delivering the material into the cells of any one of claims 8 to 15.

17. The microfluidic chip of claim 16, comprising:
a plurality of channels for implementing the method for delivering the material into the cells of any one of claims 8 to 15.

18. The microfluidic chip of claim 17, wherein the channels are defined in parallel with each other.
